# EUROPEAN PATENT APPLICATION

(11) **EP 1 873 253 A2**
(43) Date of publication of application: **02.01.2008**
(21) Application number: 07012050.6
(22) Date of filing: 20.06.2007
(51) Int. Cl.: C12P 7/06

(54) **Process for the production of ethanol from fractionated plant material with vitamin B as fermentation supplement**

(30) Priority: 29.06.2006 US 806192 P; 21.02.2007 US 708923
(71) Applicant: Cognis IP Management GmbH, 40589 Düsseldorf (DE)
(72) Inventor: Wenzel, Jon D., Cincinnati, OH 45245 (US); Oester, Dean, Cincinnati, OH 45236 (US)

(57) **Abstract**

A process for the production of ethanol including (a) forming a mash with a slurry of fractionated plant material; (b) saccharifying the mash; (c) adding to the mash a supplement selected from a crude Vitamin B source, a lipid, a vegetable oil distillate, organic nitrogen, and mixtures thereof; (d) fermenting the mash with a microorganism to form a fermentation broth; and (e) recovering ethanol from the fermentation broth is provided. A process for the production of ethanol, including (a) forming a mash with a slurry of nutrient-deficient plant material; (b) saccharifying the mash; (c) adding to the mash a supplement selected from a crude Vitamin B source, a lipid, a vegetable oil distillate, organic nitrogen, and mixtures thereof; (d) fermenting the mash with a microorganism to form a fermentation broth; and (e) recovering ethanol from the fermentation broth is also provided.

## Description

This application claims priority under 35 U.S.C. § 120 from U.S. Provisional Application No. 60/806,192, filed on June 29, 2006, the entire disclosure of which is hereby incorporated by reference.

### Background of the invention

### Field of the Invention

The invention relates generally to the production of ethanol, and more particularly, to supplemental components for increasing ethanol production from fractionated and other nutrient-poor substrates.

### Background Information

In the process of ethanol fermentation, yeast and enzymes are combined with a starchy grain or vegetable, for example, corn, to produce ethanol for beverages, fuels, and industrial uses.

Domestic fuel ethanol production predominately uses corn as the fermentation substrate in a dry grind process, which involves grinding corn and mixing with water to produce a slurry. The slurry is heated to obtain a slurry starch. The starch is liquefied, saccharified and fermented to produce ethanol. The remaining germ, fiber and protein are recoverable at the end of the process and used as an animal food product known as distillers dried grain with solubles (DDGS).

Corn fractionation technologies are available to reduce the amount of distillers dried grain with solubles and to enhance the profitability of the process through the generation of value added co-products. In a fractionation process, the germ is separated from the pericarp fiber. The endosperm fraction is subjected to fermentation, but the results are inferior in terms of the rate of ethanol production and ethanol yield when compared to whole ground corn, due to the loss of nutrients removed during fractionation that are no longer available to the yeast during fermentation.

A need remains for methods for producing increased amounts of ethanol from plant materials that have lost nutrients during fractionation processes, while reducing the amounts of less valuable by-products. A need remains also for methods for producing ethanol from nutrient-deficient plant materials.

### Summary of the invention

Briefly described, according to an aspect of the invention, in one embodiment, a process for the production of ethanol includes the steps of: (a) forming a mash with a slurry of fractionated plant material; (b) saccharifying the mash; (c) adding to the mash a supplement selected from the group consisting of a crude Vitamin B source, a lipid, a vegetable oil distillate, organic nitrogen, and mixtures thereof; (d) fermenting the mash with a microorganism to form a fermentation broth; and (e) recovering ethanol from the fermentation broth.

According to another aspect of the invention in another embodiment, a process for the production of ethanol includes the steps of: (a) forming a mash with a slurry of nutrient-deficient plant material; (b) saccharifying the mash; (c) adding to the mash a supplement selected from the group consisting of a crude Vitamin B source, a lipid, a vegetable oil distillate, organic nitrogen, and mixtures thereof; (d) fermenting the mash with a microorganism to form a fermentation broth; and (e) recovering ethanol from the fermentation broth.

### Detailed description of the invention

According to an aspect of the invention, in one embodiment, a process for the production of ethanol includes the steps of: (a) forming a mash with a slurry of fractionated plant material; (b) saccharifying the mash; (c) adding to the mash a supplement selected from the group consisting of a crude Vitamin B source, a lipid, a vegetable oil distillate, organic nitrogen, and mixtures thereof; (d) fermenting the mash with a microorganism to form a fermentation broth; and (e) recovering ethanol from the fermentation broth.

The fractionated plant material may be selected from the group consisting of corn and sorghum. The fractionated plant material includes the endosperm fraction of corn. The supplement may be a crude Vitamin B source. The crude Vitamin B source may be an extract of alfalfa, banana, potato, yeast, and mixtures thereof. The crude Vitamin B source may include B1, B2, B6, niacinamide, panthothenic acid, PABA, inositol and folic acid. The crude Vitamin B source may be added in amounts of from 10 ppm to 5% (w/w).

The supplement may also include a lipid. The supplement may include a lipid and a crude Vitamin B source. The supplement may also include a lipid and organic nitrogen, and may also include a protease. The lipid may be selected from the group consisting of fatty acid esters, esters of mono- and poly-hydric alcohols, sorbitan esters, sorbitan ester ethoxylates, alkyl phenyl ethoxylates, triglygeride ethoxylates, methyl and ethyl esters, and mixtures thereof. The lipid is added in amounts of from 50 ppm to 5000 ppm.

The supplement may also include a vegetable oil distillate. The supplement may also include a crude Vitamin B source and a vegetable oil distillate. The supplement may also include a vegetable oil distillate and organic nitrogen, and may also include a protease. The vegetable oil distillate is added in amounts of from 50 ppm to 5000 ppm.

The supplement may also include a lipid and a vegetable oil distillate.
The supplement may also include organic nitrogen, and may also include a protease.
The supplement may also include a crude Vitamin B source, a lipid, and organic nitrogen, and may also include a protease. The supplement may also include a crude Vitamin B source, a vegetable oil distillate, and organic nitrogen, and may also include a protease. The supplement may also include a crude Vitamin B source and organic nitrogen, and may also include a protease.

The microorganism may be a yeast, for example, *Saccharomyces cerevisiae.* The microorganism may be a bacteria, for example, *Escherichia coli* or *Zymomonas mobilis.*

Steps (b), (c) and (d) may occur continuously and simultaneously, and step (c) may occur simultaneously with step (d).

According to an aspect of the invention in another embodiment, a process for the production of ethanol includes the steps of: (a) forming a mash with a slurry of nutrient-deficient plant material; (b) saccharifying the mash; (c) adding to the mash a supplement selected from the group consisting of a crude Vitamin B source, a lipid, a vegetable oil distillate, organic nitrogen, and mixtures thereof; (d) fermenting the mash with a microorganism to form a fermentation broth; and (e) recovering ethanol from the fermentation broth.

The supplement may include a crude Vitamin B source. The crude Vitamin B source may be an extract of alfalfa, banana, potato, yeast, and mixtures thereof. The crude Vitamin B source may include B1, B2, B6, niacinamide, panthothenic acid, PABA, inositol and folic acid. The crude Vitamin B source may be added in amounts of from 10 ppm to 5% (w/w). The nutrient-deficient plant material may be selected from the group consisting of wheat, millet, barley, rye, biomass and mixtures thereof.

The lipid may be selected from the group consisting of fatty acid esters, esters of mono- and poly-hydric alcohols, sorbitan esters, sorbitan ester ethoxylates, alkyl phenyl ethoxylates, triglygeride ethoxylates, methyl and ethyl esters, and mixtures thereof. The lipid is added in amounts of from 50 ppm to 5000 ppm. The vegetable oil distillate is added in amounts of from 50 ppm to 5000 ppm.

The supplement may include organic nitrogen, and may also include a protease.

The microorganism may be a yeast, for example, *Saccharomyces cerevisiae.* The microorganism may be a bacteria, for example, *Escherichia coli* or *Zymomonas mobilis.*

Steps (b), (c) and (d) may occur continuously and simultaneously, and step (c) may occur simultaneously with step (d).

### Definitions

As used herein, the terms "comprises", "comprising", "includes", "including", "has", "having", or any other variation thereof, are intended to cover non-exclusive inclusions. For example, a process, method, article or apparatus that comprises a list of elements is not necessarily limited to only those elements but may include other elements not expressly listed or inherent to such process, method, article, or apparatus. In addition, unless expressly stated to the contrary, the term "or" refers to an inclusive "or" and not to an exclusive "or". For example, a condition A or B is satisfied by any one of the following: A is true (or present) and B is false (or not present); A is false (or not present) and B is true (or present); and both A and B are true (or present).

The terms "a" or "an" as used herein are to describe elements and components of the invention. This is done merely for convenience and to give a general sense of the invention. The description herein should be read to include one or at least one and the singular also includes the plural unless indicated to the contrary.

The term "mash" refers to ground plant material mixed with water to produce a slurry.

The term "fractionated plant material" refers to plant material capable of being fractionated, and includes, but is not limited to corn and sorghum, for example, milo. Fractionated plant material includes a germ, pericarp fiber, and endosperm. The endosperm is used in the fermentation process.

The term "nutrient-deficient plant material" refers to plant material which may lack necessary nutrients for completion of a fermentation process, and includes, but is not limited to wheat, millet, barley, rye and biomass, for example, switchgrass.

The term "microorganism" refers to a catalyst for the fermentation process, and includes, but is not limited to yeasts and bacteria.

The term "fermentation" refers to a chemical pathway that provides a fermented substance. Broadly defined, "fermentation" is an enzymatically controlled transformation of an organic compound. In ethanol fermentation (using yeasts and enzymes and possibly other microorganisms), there is an anerobic breakdown of an energy-rich compound (carbohydrate to carbon dioxide and alcohol, or to an organic acid). Specifically, an ionized carboxyl group is removed from pyruvate (formed from a molecule of glucose by glycolysis) to generate a molecule of carbon dioxide, and the resulting molecule (acetaldehyde) accepts a hydrogen ion to form ethanol.

The term glycolysis refers to the conversion of glucose (formed in the mash) by glycolitic enzymes to pyruvic acid.

The term "lipid" includes, but is not limited to fatty acid esters (including, but not limited to methyl and ethyl esters), esters of mono- and poly-hydric alcohols, sorbitan esters, sorbitan ester ethoxylates, alkyl phenyl ethoxylates, and triglygeride ethoxylates, and mixtures thereof.

The term "vegetable oil distillate" (VOD) means the resulting chemical fatty streams produced by the high temperature, low pressure, steam stripping of vegetable oils in the process of vegetable oil refining. A VOD contains unsaponifiables and fatty materials.

In a whole corn kernel dry grind process for forming a mash, the entire corn kernel or other starchy grain is first ground into flour (referred to as "meal") and processed without separating the various parts of the grain. The meal is slurried with water to form a mash. Enzymes added to the mash convert the starch to dextrose, a simple sugar. Ammonia is added for pH control and as a nutrient for the yeast.

In a fractionated ethanol mash, corn grits (endosperm pieces with germ and pericarp fiber removed by fractionation) are mixed with water at 60°C to obtain a slurry having 25% solids. The slurry is liquefied using 0.13% (w/w) alpha-amylase at 90°C for 90 minutes. The liquefied mash is cooled to 30°C and pH is adjusted to 4.2 using 10N sulfuric acid. The liquefied mash is saccharified by adding 0.1% glucoamylase (w/w solids basis), to which 0.008 g/g (dry solids) ammonium sulfate is added to provide free amino nitrogen. Simultaneously, the mash is inoculated with active dry yeast at 0.035 g/g dry solids. It should be understood that although fractionated corn is exemplified, this aspect of the invention is not limited to corn, and may include sorghum, for example, milo, and other suitable plant material sources.

In a fractionation process, the germ is separated from the pericarp fiber. These components may be used as a source of additional products, for example, oil from the germ, and fiber oil from the pericarp and endosperm fiber. Lipids in corn are generally present in the germ and the aleurone layer (below the pericarp). The endosperm resulting from a fractionation process and subjected to fermentation may lack nutrients, ions, vitamins, and organic nitrogen. The nutrients may include, but are not limited to oxygen, phosphate, sulfate, magnesium calcium, zinc, and other ions, vitamins, and organic nitrogen. Although these nutrients, ions and vitamins may be present in the mash, the concentrations of the individual nutrients may be insufficient, and result in lower rates of fermentations, and hence, lower amounts of ethanol produced.

Advantageously, in one aspect of the invention, it has been found that the addition of a crude vitamin B source to the mash results in increased ethanol production from fractionated plant material or nutrient-deficient plant material. In addition, by using fractionated plant material, for example, the endosperm of corn, there is an additional advantage because less waste product remains. The fractionation process is intended to recover value added co-products (germ and pericarp fiber) which are advantageously no longer needed for the production of ethanol according to an aspect of the invention.

Advantageously, in another aspect of the invention, the addition of a lipid or mixture of lipids to the mash keeps a yeast (or bacteria) metabolically active for as long as possible to obtain increased yield of ethanol. The addition of lipids helps stabilize the membrane of the yeast. The addition of lipids also improves ethanol tolerance. Surprisingly, it was found that the use of ethoxylated compounds, which may impart resilience to the deleterious effects of the increasing alcohol concentration, were useful in an aspect of the invention. By adding lipids to the mash, the production of ethanol, which is toxic to yeast, is increased. The addition of lipids may also replace the lost nutrients from the fractionation process, which are necessary for the fermentation process, or provide the necessary nutrients to a nutrient-deficient plant material.

In another aspect of the invention, the addition of a vegetable oil distillate advantageously provides the necessary nutrients which are unavailable when using a fractionated plant material, or when the plant material is nutrient-deficient.

Although other suitable sources of crude Vitamin B may be added to the mash, a suitable crude Vitamin B source that includes organic nitrogen rich in short chain peptides is AGRIMUL® NSV20, available from Cognis Corporation, Ohio. AGRIMUL® NSV20 is derived from a forage crop and is available in an extract form. Forage crops include alfalfa, clovers, and vetch. AGRIMUL® NSV20 includes biotin, choline, folic acid, inositol, niacin, pantothenic acid, Vitamins B1, B2 and B6, and p-amino benzoic acid (PABA). A typical loading for AGRIMUL® NSV20 is 500 ppm in the mash. Other suitable sources for crude Vitamin B include extracts of banana, potato, yeast, and mixtures thereof.

A suitable yeast for use in the invention includes, but is not limited to *Saccharomyces cervisiae,* which are available commercially. A suitable bacteria includes, but is not limited to *Escherichia coli.*

Suitable VODs are available throughout the world from a number of commercial suppliers.

A suitable protease available from Genencor is GC106. The protease source is *Aspergillus niger.* A typical loading for the protease is 500-1000 ppm.

Unless otherwise defined, all technical and scientific terms used herein have the same meaning as commonly understood by one of ordinary skill in the art to which the invention belongs. Although methods and materials similar or equivalent to those described herein can be used in the practice or testing of the invention, suitable methods and materials are described below. In addition, the materials, methods and examples are illustrative only and are not intended to be limiting.

The following paragraphs describe some embodiments of the present invention:
1. A process for the production of ethanol, comprising the steps of:
   (a) forming a mash with a slurry of fractionated plant material;
   (b) saccharifying the mash;
   (c) adding to the mash a supplement selected from the group consisting of a crude Vitamin B source, a lipid, a vegetable oil distillate, organic nitrogen, and mixtures thereof;
   (d) fermenting the mash with a microorganism to form a fermentation broth; and
   (e) recovering ethanol from the fermentation broth.
2. The process according to embodiment no. 1, wherein the supplement of step (c) comprises a crude Vitamin B source.
3. The process according to embodiment no. 1, wherein the supplement of step (c) comprises a crude Vitamin B source and a lipid.
4. The process according to embodiment no. 1, wherein the supplement of step (c) comprises a crude Vitamin B source, a lipid, and organic nitrogen.
5. The process according to embodiment no. 4, wherein the supplement further comprises a protease.
6. The process according to embodiment no. 1, wherein the supplement of step (c) comprises a crude Vitamin B source and a vegetable oil distillate.
7. The process according to embodiment no. 1, wherein the supplement of step (c) comprises a crude Vitamin B source, a vegetable oil distillate, and organic nitrogen.
8. The process according to embodiment no. 7, wherein the supplement further comprises a protease.
9. The process according to embodiment no. 1, wherein the supplement of step (c) comprises a crude Vitamin B source and organic nitrogen.
10. The process according to embodiment no. 9, wherein the supplement further comprises a protease.
11. The process according to embodiment no. 1, wherein the supplement of step (c) comprises a lipid.
12. The process according to embodiment no. 1, wherein the supplement of step (c) comprises a lipid and organic nitrogen.
13. The process according to embodiment no. 12, wherein the supplement further comprises a protease.
14. The process according to embodiment no. 1, wherein the supplement of step (c) comprises a vegetable oil distillate.
15. The process according to embodiment no. 1, wherein the supplement of step (c) comprises a vegetable oil distillate and organic nitrogen.
16. The process according to embodiment no. 15, wherein the supplement further comprises a protease.
17. The process according to embodiment no. 1, wherein the supplement of step (c) comprises a lipid and a vegetable oil distillate.
18. The process according to embodiment no. 1, wherein the supplement of step (c) comprises organic nitrogen.
19. The process according to embodiment no. 18, wherein the supplement further comprises a protease.
20. A process for the production of ethanol, comprising the steps of:
   (a) forming a mash with a slurry of nutrient-deficient plant material;
   (b) saccharifying the mash;
   (c) adding to the mash a supplement selected from the group consisting of a crude Vitamin B source, a lipid, a vegetable oil distillate, organic nitrogen, and mixtures thereof;
   (d) fermenting the mash with a microorganism to form a fermentation broth; and
   (e) recovering ethanol from the fermentation broth.
21. The process according to embodiment no. 20, wherein the supplement of step (c) comprises a crude Vitamin B source.
22. The process according to embodiment no. 20, wherein the supplement of step (c) comprises organic nitrogen.
23. The process according to embodiment no. 22, wherein the supplement further comprises a protease.

### Examples

### Example 1

In a fermentation process in a suitable vessel, suitable mediums for microorganisms may include the following components and amounts, although other suitable mediums are available:

### Synthetic medium (Saccharomyces cerevisiae)

| Component | Amount (g/L) |
|---|---|
| Glucose | 100 |
| Ammonium sulphate | 5.19 |
| Potassium dihydrogen phosphate | 1.53 |
| Magnesium sulphate - 7H20 | 0.55 |
| Calcium chloride | 0.13 |

Plus trace amounts of Boric acid, Co, Cu, Zi, Mn, I, Fe, Al, Biotin, Pantothenate, Inositol, Thiamin, Pyridoxine, PABA, Nicotinic acid

### Semi-synthetic medium (Zymomonas mobilis)

| Component | Amount (g/L) |
|---|---|
| Glucose | 100 |
| Yeast Extract | 5 |
| Ammonium sulphate | 1 |
| Potassium dihydrogen phosphate | 1 |
| Magnesium sulphate - 7H20 | 0.5 |

### Ethanol Fermentation Conditions

| Parameter | Broad Range | Preferred Value |
|---|---|---|
| Temperature | 28 - 35°C | 30°C |
| pH | 2.4-8.6 | 4.5 |

Using the above-defined parameters, the following components may be added to a mash of fractionated corn to obtain increased ethanol production:
(1) a crude Vitamin B source;
(2) a lipid;
(3) a vegetable oil distillate;
(4) a crude Vitamin B source and a lipid;
(5) a crude Vitamin B source and a vegetable oil distillate;
(6) a crude Vitamin B source, a lipid, and a vegetable oil distillate;
(7) a lipid and a vegetable oil distillate;
(8) any of the above and an organic nitrogen source; and
(9) an organic nitrogen source, and a protease added to any of the above components which include organic nitrogen.

The above-defined parameters may be used alternatively with a nutrient-deficient plant material in combination with (1) through (9) above. When a nutrient-deficient plant material includes biomass, the mash may be treated with a cellulase to enhance the production of ethanol.

It should be understood that the above components may be added throughout the fermentation process in discrete shots or by continuous or semi-continuous fed-batch operation. The nutrients needed for the fermentation process may be absent based on the starting plant material, or become lost during the active fermentation process. For example, nutrients may be depleted, or the viability of the yeast or bacterium may decline during the process. To obtain the greatest yield, nutrients are added to continue the process or increase the rate or reaction.

### Example 2

In this Example, four lipid supplements (A-D) were tested against a control.
A: 25% of a waste stream from a tocopherol refining process starting with VOD and 75% of a rapeseed oil ethoxylate;
B: 25% of a waste stream from a tocopherol refining process starting with VOD and 75% of a polyethylene glycol (PEG) 400 monostearate;
C: VOD;
D: Sorbitan monooleate; and
E: Control (no lipids).

The lipid supplements were added, at loading, of 500 ppm and 1000 ppm. Sample points were obtained at 0, 24, 48 and 72 hours. The fermentation procedures were conducted in a 200 mL lab apparatus. The vitamin additives included regular B vitamins, and a crude B-Vitamin and an organic nitrogen source available from Cognis Corp. of Ohio under the tradename of Agrimul® NSV20. The B-vitamins were added at 500 ppm.

The results of the experiments are found in Tables 1 and 2.

**Table 1**

| Ethanol concentrations for lipid supplements at 500 ppm level. | | | | |
|---|---|---|---|---|
| Ethanol concentration (% v/v) | | | | |
| Time (hr) | 0 | 24 | 48 | 72 |
| No B-vitamins/Organic nitrogen | | | | |
| A | 0.16 | 6.33 | 8.46 | 13.01 |
| B | 0.20 | 6.62 | 12.39 | 13.48 |
| C | 0.14 | 5.07 | 8.53 | 13.83 |
| D | 0.12 | 6.10 | 9.94 | 13.21 |
| E | 0.18 | 6.35 | 10.70 | 12.90 |

| B-vitamins | | | | |
|---|---|---|---|---|
| A | 0.24 | 7.28 | 12.65 | 15.14 |
| B | 0.14 | 6.72 | 12.80 | 15.27 |
| C | 0.18 | 7.93 | 12.43 | 14.99 |
| D | 0.32 | 7.50 | 13.12 | 14.88 |
| E | 0.18 | 7.89 | 9.88 | 15.14 |

| Organic nitrogen with protease | | | | |
|---|---|---|---|---|
| A | 0.18 | 7.48 | 13.49 | 15.22 |
| B | 0.22 | 8.43 | 12.03 | 14.96 |
| C | 0.16 | 8.21 | 13.25 | 15.30 |
| D | 0.17 | 8.77 | 13.98 | 15.53 |
| E | 0.20 | 8.63 | 13.81 | 15.57 |

**Table 2**

| Ethanol concentrations for lipid supplements at 1000 ppm level. | | | | |
|---|---|---|---|---|
| Ethanol (% v/v) | | | | |
| Time (hr) | 0 | 24 | 48 | 72 |
| No B-vitamins/Organic nitrogen | | | | |
| A | 0.10 | 6.60 | 10.77 | 13.21 |
| B | 0.11 | 6.70 | 10.92 | 13.36 |
| C | 0.11 | 6.65 | 10.36 | 13.53 |
| D | 0.10 | 6.71 | 10.70 | 12.59 |
| E | 0.11 | 6.73 | 10.75 | 13.18 |

| B-vitamins | | | | |
|---|---|---|---|---|
| A | 0.10 | 7.59 | 12.39 | 14.87 |
| B | 0.10 | 7.29 | 12.84 | 15.04 |
| C | 0.10 | 7.65 | 12.43 | 14.80 |
| D | 0.11 | 7.81 | 13.13 | 15.18 |
| E | 0.10 | 7.96 | 13.83 | 15.65 |

| Organic nitrogen with protease | | | | |
|---|---|---|---|---|
| A | 0.11 | 8.71 | 14.58 | 15.47 |
| B | 0.09 | 8.31 | 14.47 | 15.48 |
| C | 0.10 | 8.48 | 13.55 | 15.66 |
| D | 0.09 | 8.39 | 14.07 | 15.64 |
| E | 0.11 | 8.70 | 15.04 | 15.63 |

As illustrated in the above Tables 1 and 2, the unexpected result was the substantial improvement in ethanol yield when comparing the control treatment (E) to the result of adding either B-Vitamins or the crude B-Vitamin/Organic Nitrogen source (Agrimul® NSV20) plus protease versus the "No B-Vitamin/Organic Nitrogen" subset. An improvement in ethanol yield also can be observed within the "No B-vitamin/Organic Nitrogen" subset as a result of adding 25% of a waste stream from a tocopherol refining process starting with VOD and 75% of a polyethylene glycol (PEG) 400 monostearate (B) or VOD (C) when compared to the control (E) with no additions.

The glucose concentration in these experiments was also measured. The results are found in Tables 3 and 4 below.

**Table 3**

| Glucose concentrations for lipid supplements at 500 ppm level. | | | | |
|---|---|---|---|---|
| Glucose concentration (% w/v) | | | | |
| Time (hr) | 0 | 24 | 48 | 72 |
| No B-vitamins/Organic nitrogen | | | | |
| A | 3.71 | 5.78 | 5.16 | 4.27 |
| B | 4.25 | 5.87 | 3.78 | 3.09 |
| C | 4.21 | 4.39 | 4.01 | 2.69 |
| D | 3.71 | 5.35 | 5.40 | 3.25 |
| E | 4.36 | 6.18 | 6.52 | 3.91 |

| B-vitamins | | | | |
|---|---|---|---|---|
| A | 4.31 | 3.44 | 3.38 | 0.17 |
| B | 2.85 | 3.11 | 3.12 | 0.22 |
| C | 3.68 | 3.34 | 3.04 | 0.13 |
| D | 4.77 | 3.43 | 2.85 | 0.21 |
| E | 3.90 | 4.02 | 2.73 | 0.57 |

| Organic Nitrogen with protease | | | | |
|---|---|---|---|---|
| A | 3.56 | 2.72 | 1.28 | 0.00 |
| B | 3.71 | 2.95 | 3.20 | 0.00 |
| C | 3.94 | 2.99 | 1.60 | 0.00 |
| D | 3.65 | 3.36 | 1.47 | 0.00 |
| E | 3.73 | 3.13 | 1.96 | 0.00 |

**Table 4**

| Glucose concentrations for lipid supplements at 1000 ppm level. | | | | |
|---|---|---|---|---|
| Glucose concentration (% w/v) | | | | |
| Time (hr) | 0 | 24 | 48 | 72 |

| No B-vitamins/Organic nitrogen | | | | |
|---|---|---|---|---|
| A | 8.52 | 7.01 | 5.51 | 3.09 |
| B | 8.77 | 6.95 | 5.37 | 2.93 |
| C | 8.14 | 7.45 | 5.08 | 2.76 |
| D | 6.74 | 7.19 | 5.44 | 2.89 |
| E | 5.72 | 7.58 | 5.79 | 3.20 |

| B-vitamins | | | | |
|---|---|---|---|---|
| A | 7.57 | 5.37 | 3.43 | 0.94 |
| B | 7.57 | 5.46 | 2.80 | 0.49 |
| C | 7.91 | 5.64 | 3.43 | 0.92 |
| D | 8.22 | 5.34 | 2.31 | 0.35 |
| E | 8.37 | 6.34 | 1.37 | 0.02 |

| Organic Nitrogen with protease | | | | |
|---|---|---|---|---|
| A | 7.04 | 4.64 | 0.82 | 0.00 |
| B | 8.24 | 4.51 | 0.93 | 0.00 |
| C | 7.25 | 4.71 | 1.33 | 0.01 |
| D | 8.12 | 5.00 | 1.24 | 0.00 |
| E | 10.12 | 6.28 | 0.26 | 0.00 |

As illustrated in the above Tables 3 and 4, the remarkable ethanol yield improvements of Tables 1 and 2 are corroborated by the substantial improvement in glucose utilization that can be seen in the control treatment (E) as the result of adding either B-Vitamins or the crude B-Vitamin/Organic Nitrogen source (Agrimul® NSV20) plus protease when compared to the "No B-Vitamin/Organic Nitrogen" subset. The improvement in glucose utilization also can be observed within the "No B-vitamin/ Organic Nitrogen" subset as a result of adding 25% of a waste stream from a tocopherol refining process starting with VOD and 75% of a polyethylene glycol (PEG) 400 monostearate (B) or VOD (C) when compared to the control (E) with no additions.

The invention has been described with reference to specific embodiments. One of ordinary skill in the art, however, appreciates that various modifications and changes can be made without departing from the scope of the invention as set forth in the claims. For example, various plant materials have been described, but alternative sources may also be used. In addition, the process is used in endosperm fermentations but whole kernel, dry grind fermentations may also be suitable. Accordingly, the specification and Examples are to be regarded in an illustrative manner, rather than a restrictive view and all such modifications are intended to be included within the scope of the invention.

Benefits, other advantages, and solutions to problems have been described above with regard to specific embodiments. The benefits, advantages, solutions to problems and any element(s) that may cause any benefit, advantage, or solution to occur or become more pronounced are not to be construed as a critical, required, or essential feature or element of any or all of the claims.

## Claims

1. A process for the production of ethanol, comprising the steps of:
(a) forming a mash with a slurry of fractionated plant material;
(b) saccharifying the mash;
(c) adding to the mash a supplement selected from the group consisting of a crude Vitamin B source, a lipid, a vegetable oil distillate, organic nitrogen, and mixtures thereof;
(d) fermenting the mash with a microorganism to form a fermentation broth; and
(e) recovering ethanol from the fermentation broth.

2. The process according to Claim 1, wherein the supplement of step (c) comprises a crude Vitamin B source.

3. The process according to Claim 1, wherein the supplement of step (c) comprises a crude Vitamin B source and a lipid.

4. The process according to Claim 1, wherein the supplement of step (c) comprises a crude Vitamin B source, a lipid, and organic nitrogen.

5. The process according to Claim 4, wherein the supplement further comprises a protease.

6. The process according to Claim 1, wherein the supplement of step (c) comprises a crude Vitamin B source and a vegetable oil distillate.

7. The process according to Claim 1, wherein the supplement of step (c) comprises a crude Vitamin B source, a vegetable oil distillate, and organic nitrogen.

8. The process according to Claim 7, wherein the supplement further comprises a protease.

9. The process according to Claim 1, wherein the supplement of step (c) comprises a crude Vitamin B source and organic nitrogen.

10. The process according to Claim 9, wherein the supplement further comprises a protease.
